# EUROPEAN PATENT SPECIFICATION

(11) **EP 3 501 534 B1**
(45) Date of publication and mention of the grant of the patent: **29.11.2023**
(21) Application number: 18209661.0
(22) Date of filing: 26.10.2009
(51) Int. Cl.: A61K 39/095

(54) **VACCINE COMPOSITIONS COMPRISING A MUTATED FACTOR H BINDING PROTEIN**
IMPFSTOFFZUSAMMENSETZUNGEN MIT EINEM MUTIERTEN FAKTOR-H-BINDENDEN PROTEIN
COMPOSITIONS DE VACCIN COMPRENANT UNE PROTÉINE DE LIAISON DU FACTEUR H MUTÉE

(30) Priority: 25.10.2008 GB 0819633
(43) Date of publication of application: 26.06.2019
(62) Divisional of application: 09753188.3
(73) Proprietor: IP2IPO Innovations Limited, London N1C 4AG (GB)
(72) Inventor: EXLEY, Rachel, London SW7 2AZ (GB); TANG, Christoph, London SW7 2AZ (GB); LEA, Susan, M., Oxford OX1 3RE (GB)
(74) Representative: Barker Brettell LLP

(56) References cited:
- EP-B1- 2 470 204
- WO-A2-2006/081259
- WO-A2-2008/001224
- MASIGNANI V ET AL: "Vaccination against Neisseria meningitidis using three variants of the lipoprotein GNA1870", THE JOURNAL OF EXPERIMENTAL MEDICINE, ROCKEFELLER UNIVERSITY PRESS, UNITED STATES, vol. 197, no. 6, 17 March 2003 (2003-03-17), pages 789-799, XP002286107, ISSN: 0022-1007
- BEERNINK PETER T ET AL: "Prevalence of factor H-binding protein variants and NadA among meningococcal group B isolates from the United States: Implications for the development of a multicomponent group B vaccine", JOURNAL OF INFECTIOUS DISEASES, UNIVERSITY OF CHICAGO PRESS, CHICAGO, IL, vol. 195, no. 10, 15 May 2007 (2007-05-15) , pages 1472-1479, XP008108299, ISSN: 0022-1899
- WELSCH ET AL: "A novel mechanism for complement-mediated killing of encapsulated Neisseria meningitidis elicited by monoclonal antibodies to factor H-binding protein (genome-derived Neisserial antigen 1870)", MOLECULAR IMMUNOLOGY, PERGAMON, GB, vol. 44, no. 1-3, 30 October 2006 (2006-10-30), page 256, XP005840301, ISSN: 0161-5890
- STEVEN JOHNSON ET AL: "Design and Evaluation of Meningococcal Vaccines through Structure-Based Modification of Host and Pathogen Molecules", PLOS PATHOGENS, vol. 8, no. 10, 25 October 2012 (2012-10-25), page e1002981, XP055129501, DOI: 10.1371/journal.ppat.1002981

## Description

The present invention relates to immunogenic compositions for use in eliciting immune responses to pathogenic organisms, and in particular, to immunogenic compositions capable of eliciting protective immune responses.

*Neisseria meningitidis* (meningococcus) is an encapsulated gram-negative diplococcus bacterium that inhabits the nasopharynx of humans. Carriage rates in the general population are usually around 10%. The complex host-pathogen relationship is usually of a commensal nature. Occasionally, however, meningococcal carriage can lead to invasive disease. This phenomenon is usually associated with strains belonging to a small number of hypervirulent clonal lineages (Caugant, D. A. et al. (1987) J Bacteriol 169:2781-2792). *N. meningitidis* is the leading cause of pyogenic meningitis worldwide and is the only bacterium capable of generating outbreaks of meningitis and septicaemia. Attack rates vary between 1-3 per 10⁵ population, depending on the endemic or epidemic prevalence of disease in any one geographical location. There is a need, therefore, to develop preventative and therapeutic strategies to reduce the incidence, mortality and morbidity of invasive meningococcal disease.

The *Neisseria meningitidis* organism is sensitive to several front line antibiotics, however, despite this, a significant number of patients diagnosed with meningococcal infection die of overwhelming disease or suffer serious complications. Mortality rates vary from 2-3% in cases of uncomplicated meningitis to 50% or more in cases of septic shock (Cartwright, K. A. and D. A. Ala'Aldeen (1997) J Infect 34:15-19).

Current licensed vaccines are based on the polysaccharide capsule that surrounds that bacterial outer membrane, and therefore only offer protection against a subset of strains which express the corresponding capsule. Additionally, there are no broadly effective vaccines against serogroup B *Neisseria meningitidis,* the most common cause of meningococcal disease in wealthy countries. This is because the serogroup B capsule consists of a polymer of sialic acid which is structurally related to a modification of a human neural cell adhesion molecule expressed in infancy and during embryonic development. There is a concern that immunisation with this antigen would cause autoimmune responses.

WO 2006/081259 relates to GNA1870-based vesicle vaccines for broad spectrum protection against diseases caused by *Neisseria meningitidis.*

The present invention relates to novel compositions, and in particular novel immunogenic compositions, comprising a modified factor H binding protein, and to the use of these compositions to elicit an immune response against *Neisseria meningitidis.*

One aim of this invention is to provide one or more compositions which can be used to elicit a protective immune response against *Neisseria meningitidis.*

According to a first aspect, the present invention provides an immunogenic composition for use in the treatment or prevention of infection or disease caused by *Neisseria meningitidis* and/or *Neisseria gonorrhoeae,* comprising at least one modified factor H binding protein, wherein the composition is capable of eliciting an immune response when administered to a human or non-human animal, wherein the modified factor H binding protein has an amino acid sequence having at least 70% sequence identity to the amino acid sequence of Seq ID No:1, and has an alanine at position number 283 as defined in Seq ID No: 1 instead of glutamic acid, which results in no factor H binding, or at least 5 fold less factor H binding compared with the factor H binding protein of Seq ID No:1.

Preferably reference herein to a factor H binding protein refers to the factor H binding protein from *Neisseria meningitidis* or *Neisseria gonorrhoeae.*

*Neisseria meningitidis* subverts the immune response of a host organism by mimicking the host. *Neisseria meningitidis* uses protein, in the form of the factor H binding protein, instead of charged-carbohydrate chemistry to recruit the host complement regulator, factor H.

In healthy individuals, the activation of complement is precisely controlled through membrane-bound and soluble plasma-regulatory proteins including factor H (fH). Factor H is a 155 kDa protein composed of twenty domains (termed complement control protein repeats, or CCPs). Several pathogens have adapted to avoid complement-mediated killing by sequestering Factor H to their surface

As discussed above, *Neisseria meningitidis* is a human-adapted pathogen of global importance as a leading cause of bacterial meningitis and septic shock (Stephens et al. (2007) Lancet 369, 2196-210). Due to Neisserial strain variation, the vaccines currently available for meningococcal disease do not provide broad based protection and are therefore of limited use. One approach has been to use the factor H binding protein (known as fHbp, GNA1870, or R2086) as an antigen in a vaccine composition. The factor H binding protein is a 27 kDa surface lipoprotein, universally present on the surface of *N. meningitidis* which elicits protective bactericidal antibodies (Fletcher et al. (2004) Infect Immun 72, 2088-100; Masignani et al. (2003) J Exp Med 197, 789-99). Factor H binding protein serves to recruit the negative regulator of complement, factor H, to the bacterial surface and contributes to the ability of the meningococcus to avoid innate immune responses by inhibiting complement-mediated lysis in human plasma (Madico et al. (2006) J Immunol 177, 501-10; Schneider et al. (2006) J Immunol 176, 7566-75).

In this invention at least one modified factor H binding protein is used as the antigen. The protein is modified to prevent, or reduce, binding of factor H to the protein. The binding of factor H to the factor H binding protein following administration of the factor H binding protein in a vaccine formulation to a subject is believed to impair the success of the vaccine. First, the presence of factor H on the factor H binding protein may limit recognition of critical epitopes on the factor H binding protein by the host immune system. Antibodies generated against these epitopes may be both bactericidal and inhibit factor H binding to bacteria rendering them sensitive to complement mediated lysis. Second, immune responses against the factor H binding protein may also elicit responses against bound factor H, in a manner similar to a hapten. This may lead to a potential, unwanted, autoimmune response. Finally, complement activation is involved in immune responses as complement proteins have adjuvant activity. Reduction of complement activation at the site of immunisation by recruitment of factor H could impair the level of immunity obtained following vaccination.

Preferably the binding of factor H to the modified factor H binding protein at least 10 fold less, preferably at least two orders of magnitude less, than the binding of factor H to the wild type factor H binding protein. Preferably the reduction in binding is measured using analyte at a concentration of about 50nM. A reduction in binding of this order would be considered a significant reduction.

In addition to the modifications to the factor H binding protein made to prevent or inhibit factor H binding, the protein may also include other mutations which do not affect the ability of the protein to act as an immunogen against infection by *N. meningitidis.* For example, other conservative amino acids changes may be made to the protein. Similary, insertions or deletions to the protein may be made which do not affect the immunogenicity of the protein.

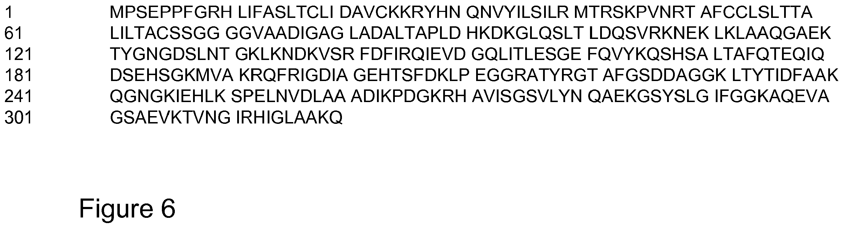

Preferably, the modified factor H binding protein has at least 80%, 85%, 90%, 95% or more sequence identity with the sequence of Figure 6.

Percentage sequence identity is defined as the percentage of amino acids in a sequence that are identical with the amino acids in a provided sequence after aligning the sequences and introducing gaps if necessary to achieve the maximum percent sequence identity. Alignment for the purpose of determining percent sequence identity can be achieved in many ways that are well known to the man skilled in the art, and include, for example, using BLAST (National Center for Biotechnology Information Basic Local Alignment Search Tool).

Variations in percent identity may be due, for example, to amino acid substitutions, insertions or deletions. Amino acid substitutions may be conservative in nature, in that the substituted amino acid has similar structural and/or chemical properties, for example the substitution of leucine with isoleucine is a conservative substitution.

An immunogenic composition is a composition that is capable of eliciting an immune response to at least one modified factor H binding protein when the composition is administered to a subject. Preferably the subject is a human or non-human mammal.

Preferably the immune response elicited by the composition of the invention affects the ability of *N. meningitidis* to infect an immunized human. Preferably the ability of *N. meningitidis* to infect a human immunised with the composition of the invention is impeded or prevented. This may be achieved in a number of ways. The immune response elicited may recognise and destroy *N. meningitidis.* Alternatively, or additionally, the immune response elicited may impede or prevent replication of *N. meningitidis.* Alternatively, or additionally, the immune response elicited may impede or prevent N. *meningitidis* causing disease in the human or non-human animal.

The modified factor H binding protein may be recombinantly produced (e.g. from a genetically-engineered expression system) or be a synthetic product, for example produced by *in vitro* peptide synthesis or *in vitro* translation.

The composition of the invention may also comprise a further one or more antigens, in addition to the one or more modified factor H binding proteins.

The further antigens may also be derived from *N. meningitidis* and may be capable of eliciting an immune response directed to *N. meningitidis.*

The composition may be used to elicit/produce a protective immune response when administered to a subject. The protective immune response may cause N. *meningitidis* to be killed upon infecting the subject, or it may prevent or inhibit *N. meningitidis* from replicating and/or from causing disease.

The composition may be used as a prophylactic or a therapeutic vaccine directed to *N. meningitidis.*

According to a further aspect (not forming part of the invention), there is provided a pharmaceutical composition comprising at least one modified factor H binding protein and a pharmaceutically acceptable carrier or excipient.

Preferably the pharmaceutical composition comprises a composition according to the first aspect of the invention.

Preferably the pharmaceutical composition is capable of producing a protective immune response to *N. meningitidis.*

The phrase "producing a protective immune response" as used herein means that the composition is capable of generating a protective response in a host organism, such as a human or a non-human mammal, to whom it is administered. Preferably a protective immune response protects against subsequent infection by *N. meningitidis.* The protective immune response may eliminate or reduce the level of infection by reducing replication of *N. meningitidis* or by affecting the mode of action of *N. meningitidis* to reduce disease.

Suitable acceptable excipients and carriers will be well known to those skilled in the art. These may include solid or liquid carriers. Suitable liquid carriers include water and saline. The proteins of the composition may be formulated into an emulsion or they may be formulated into biodegradable microspheres or liposomes.

The composition may further comprise an adjuvant. Suitable adjuvants will be well known to those skilled in the art, and may include Freund's Incomplete Adjuvant (for use in animals), and metal salts, such as aluminium or calcium salts.

The composition may also comprise polymers or other agents to control the consistency of the composition, and/or to control the release of the antigen/secreted protein from the composition.

The composition may also comprise other agents such as diluents, which may include water, saline, glycerol or other suitable alcohols etc; wetting or emulsifying agents; buffering agents; thickening agents for example cellulose or cellulose derivatives; preservatives; detergents, antimicrobial agents; and the like.

Preferably the active ingredients in the composition are greater than 50% pure, usually greater than 80% pure, often greater than 90% pure and more preferably greater than 95%, 98% or 99% pure. With active ingredients approaching 100% pure, for example about 99.5% pure or about 99.9% pure, being used most often.

The composition of the present invention may be used as vaccine against infections caused by *N. meningitidis.* The composition may be used as a vaccine directed to meningitis or other invasive meningococcal diseases including septicaemia or septic shock. The vaccine may be administered prophylactically to those at risk of exposure to *N. meningitidis,* and/or therapeutically to persons who have already been exposed to *N. meningitidis.*

Preferably, if the composition is used as a vaccine, the composition comprises an immunologically effective amount of antigen wherein the composition comprises at least one modified factor H binding protein. An "immunologically effective amount" of an antigen is an amount that when administered to an individual, either in a single dose or in a series of doses, is effective for treatment or prevention of infection by *N. meningitidis.* This amount will vary depending upon the health and physical condition of the individual to be treated and on the antigen. Determination of an effective amount of an immunogenic or vaccine composition for administration to an organism is well within the capabilities of those skilled in the art.

A composition according to the invention may be for oral, systemic, parenteral, topical, mucosal, intramuscular, intravenous, intraperitoneal, intradermal, subcutaneous, intranasal, intravaginal, intrarectal, transdermal, sublingual, inhalation or aerosol administration.

The composition may be arranged to be administered as a single dose or as part of a multiple dose schedule. Multiple doses may be administered as a primary immunisation followed by one or more booster immunisations.

Suitable timings between priming and boosting immunisations can be routinely determined.

A composition according to the invention may be used in isolation, or it may be combined with one or more other immunogenic or vaccine compositions, and/or with one or more other therapeutic regimes.

Compositions of the invention may be able to induce a serum bactericidal antibody responses and elicit antibodies which mediate opsonphagocytosis after being administered to a subject. These responses are conveniently measured in mice and the results are a standard indicator of vaccine efficacy.

The compositions of the invention may also, or alternatively, be able to elicit an immune response which neutralises bacterial proteins or other molecules, thereby preventing them from having their normal function and preventing or reducing disease progression without necessarily destroying the pathogenic organism/bacteria, in this case to *N. meningitidis.*

According to a further aspect, the present invention provides the use of a modified factor H binding protein for the preparation of a medicament for the treatment or prevention of infection or disease caused by Neisseria meningitidis and/or Neisseria gonorrhoeae, wherein the modified factor H binding protein is capable of eliciting an immune response when administered to a human or non-human animal having an amino acid sequence having at least 70% sequence identity to the amino acid sequence of Seq ID No:1, and having an alanine at position number 283 as defined in Seq ID No: 1 instead of glutamic acid, which results in no factor H binding, or at least 5 fold less factor H binding compared with the factor H binding protein of Seq ID No:1. The medicament may be used for the prophylactic or therapeutic vaccination of subjects against *N. meningitidis.* The medicament may be a prophylactic or a therapeutic vaccine. The vaccine may be for meningitis, septicaemia and/or septic shock caused by *N. meningitidis.*

According to a yet further aspect (not forming part of the present invention), there is provided a composition comprising one or more modified factor H binding proteins for use in generating an immune response to *N. meningitidis.* The immune response may be prophylactic or therapeutic. The composition may be for use as a vaccine.

According a still further aspect (not forming part of the present invention), there is provided a method of protecting a human or non-human animal from the effects of infection by *N. meningitidis* comprising administering to the human or non-human animal a composition according to any other aspect of the invention. The composition may be a vaccine.

According to another aspect (not forming part of the invention), there is provided a method for raising an immune response in a human or non-human animal comprising administering a pharmaceutical composition according to the invention to the human or non-human animal. The immune response is preferably protective. The method may raise a booster response in a patient that has already been primed. The immune response may be prophylactic or therapeutic.

One way to check the efficacy of a therapeutic treatment comprising administration of a composition according to the invention involves monitoring for *N. meningitidis* infection after administration of the composition. One way to check the efficacy of a prophylactic treatment comprising administration of a composition according to the invention involves monitoring immune responses to *Neisseria meningitidis* after administration of the composition.

According to another aspect (not forming part of the present invention), there is provided the use of one or more modified factor H binding proteins in the preparation of a medicament for use in the immunisation of human or non-human mammals against infection by *N. meningitidis.*

According to a further aspect the invention (not forming part of the present invention), there is provided a kit for use in inducing an immune response in an organism, comprising an immunogenic or vaccine composition according to the invention and instructions relating to administration.

In addition to their potential use as vaccines, compositions according to the invention may be useful as diagnostic reagents and as a measure of the immune competence of a vaccinee.

While all the statements of invention and preferable features discussed above refer to *N. meningitidis,* the skilled man will appreciate that they could equally apply to *N. gonorrhoeae.*

The skilled man will appreciate that any of the preferable features discussed above can be applied to any of the aspects of the invention.

Preferred embodiments of the present invention will now be described, merely by way of example, with reference to the following figures and examples.

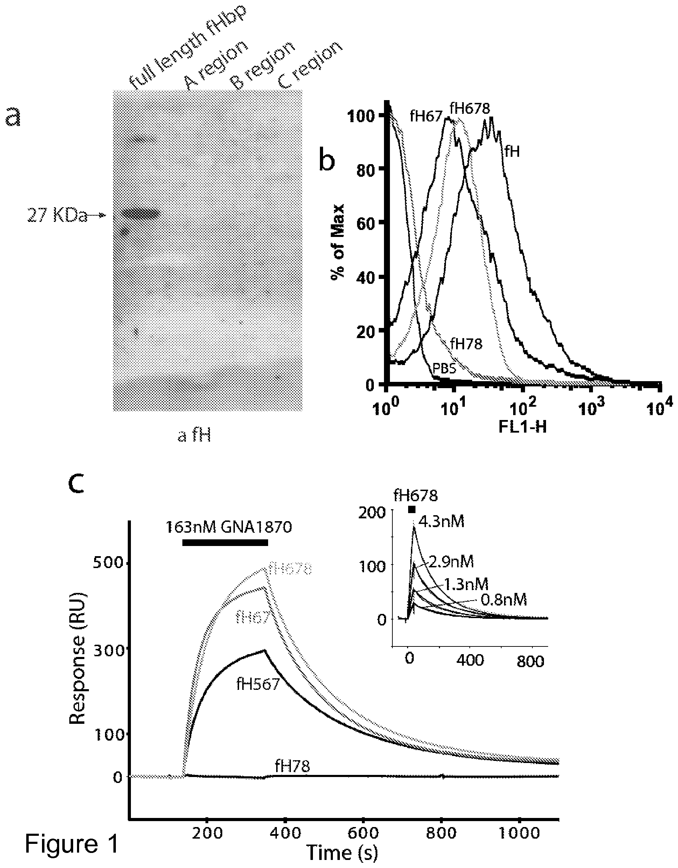

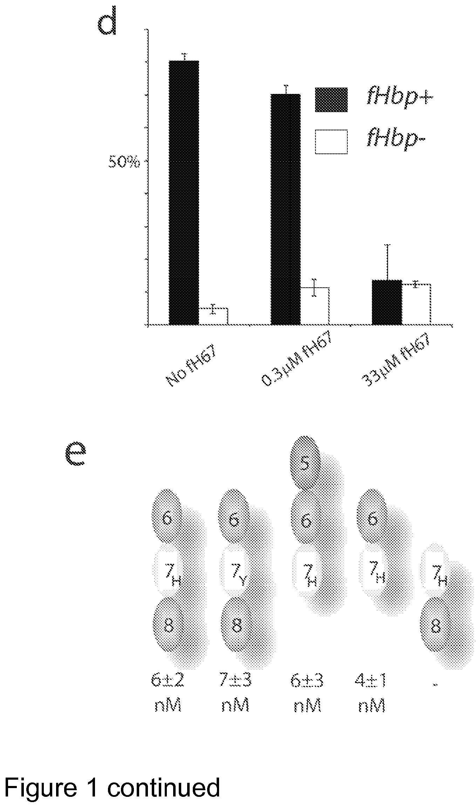

**Figures 1a** **to If** - Figure 1a to 1f demonstrate that the factor H binding protein binding site is localised to CCP6 of factor H and requires the complete extracellular portion of the factor H binding protein. **Figure la** shows Far Western analysis of factor H binding to intact factor H binding protein and truncated versions thereof (as indicated). Membranes were incubated with purified factor H which was detected with α-fH pAbs. Binding was only observed to the intact 27 kDa factor H binding protein (shown with an arrow). **Figure 1b** shows the results of FACS analysis with α-fH pAbs which detected binding between *N. meningitidis* and factor H. **Figure 1c** uses SPR results to demonstrate that the factor H binding protein is only able to bind to factor H constructs containing CCP6. The inset shows a 1:1 Langmuir fit to series of fH67 dilutions injected over a factor H binding protein surface to determine kinetic parameters. **Figure 1d** shows the results of FACS competition studies (using α-fH mAb directed against CCP5 and therefore unable to recognize the fH67 construct) which demonstrate that the short fH67 construct (between ~0.3 and 30 *µ*M) can compete away binding of full length factor H demonstrating that this construct contains the entire factor H binding protein binding site. Values shown are mean fluorescence intensity from three experiments ± s.d. **Figure 1e** shows that quantitation with SPR confirms that the presence of CCP6 is necessary and sufficient for high affinity binding to the factor H binding protein and that the common factor H polymorphism in CCP7 (402His/Tyr) does not significantly alter the affinity of fHbp binding. **Figure If** is a table illustrating the binding constants obtained using Surface Plasmon Resonance with the factor H binding protein on the surface of the chip and the factor H constructs in the fluid phase. Fits were made using BiaEvaluation 2.0 software and a 1:1 Langmuir model. Values shown are mean ± standard deviation, Chi2 are the quality of fit indicators given within BiaEvaluation 2.0.

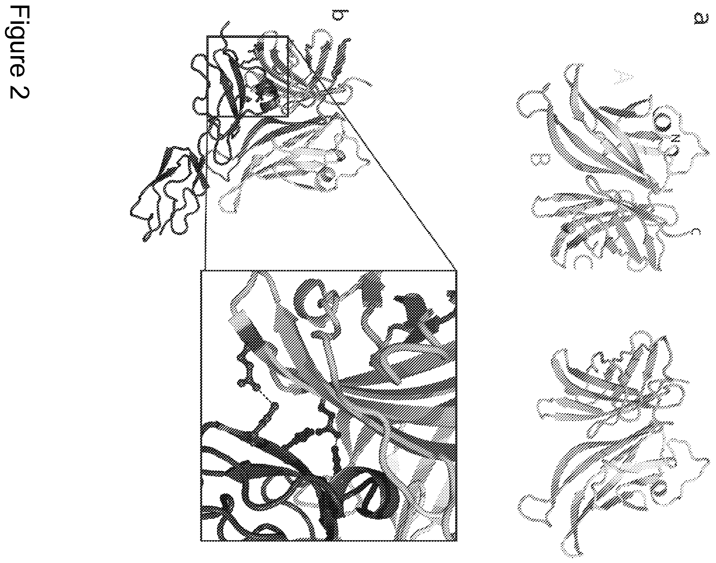

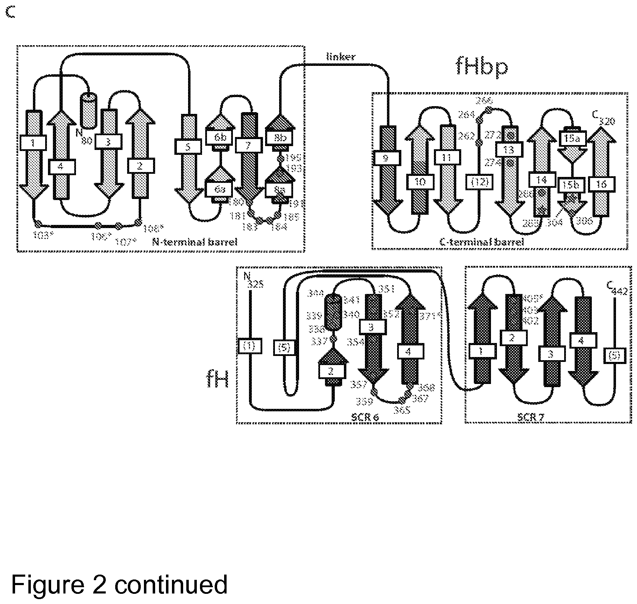

**Figures 2a to 2c** **-** illustrates the structure of the factor H binding protein and its complex with fH67. **Figure 2a** shows two views of a cartoon representation of the factor H binding protein (residues 80 to 320). The A, B and C regions are indicated. **Figure 2b** shows a cartoon of the fHbp:fH67 complex between the factor H binding protein and CCPs 6 and 7 from factor H. Side chains from both proteins involved in forming salt bridges across the interaction surface are shown as ball-and-stick representations (zoomed and reoriented in the inset box). **Figure 2c** illustrates the topology of the factor H binding protein and of fh67, the residues involved in either H-bond or salt-bridge interactions between the proteins are indicated. The solid circles indicate residues which make a hydrogen bond with their partner. The solid stars indicate residues which make a salt-bridge to their partner. The asterix indicates an interaction which is only seen in some of the independent copies of the complex structure,

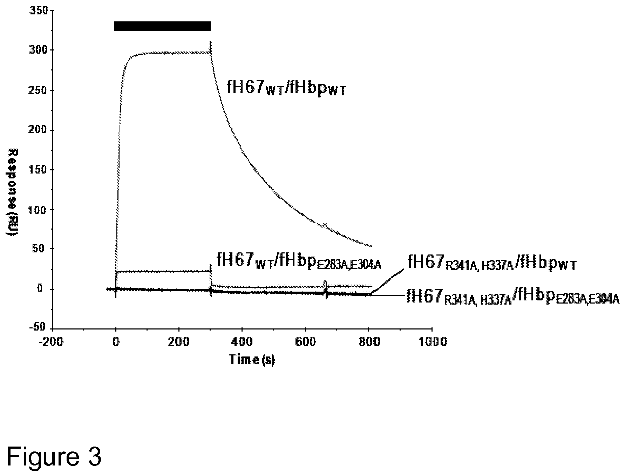

**Figure 3** - relates to the interface between the factor H binding protein and factor H, and demonstrates that site-directed mutagenesis to replace charged side-chains with alanine in both factor H and in the factor H binding protein, causes the abolition of the binding of each to the wild-type form of their partner at concentrations around the wild-type KD. The black bar indicates the time period for which the factor H analytes were injected (at 50nM, 40µl.min⁻¹) over the factor H binding protein surfaces.

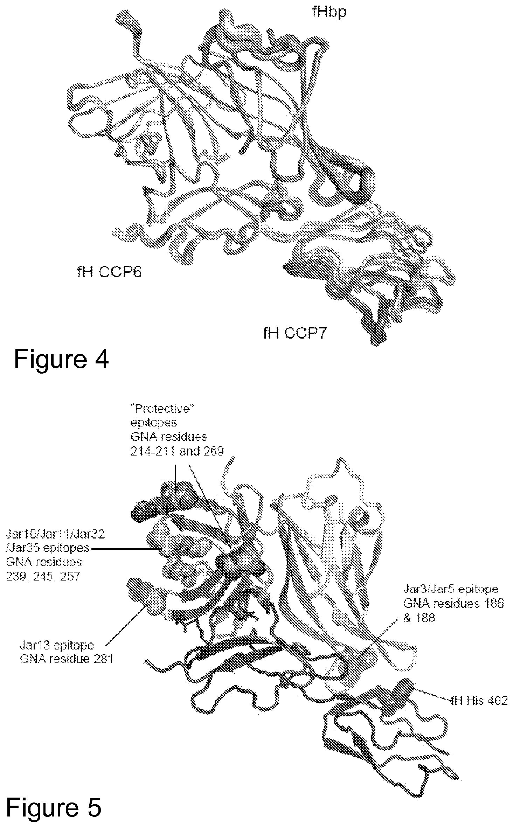

**Figure 4** - considers the flexibility in the fH67/fHbp complex using an overlay of the seven, crystallographically independent copies of the fH67/fHbp complex from the two crystal forms (four copies of the complex in the P1 form and 3 copies in the C2 form). The high degree of similarity, despite very different packing environments, indicates that the complex is biologically relevant. Rearrangement of the proteins within the complex is limited to a small amount of movement in the position of CCP7 of fH67 relative to CCP6 and factor H binding protein. The loops of the factor H binding protein which pack closest to CCP7 are also relatively mobile as indicated by their higher temperature factors (displayed here as a "thicker" putty representation).
**Figure 5** - shows the location of antibody epitopes mapped on the surface of the factor H binding protein. Residues in the protective epitopes deCCPibed in 12,20,21 are shown in CPK representations (Glu 211, Arg 214 and Arg 269) coloured by element (C green, N blue, O red). Epitopes for antibodies which block factor H binding shown as CPK coloured according to colouring of the factor H binding protein (region "A" yellow, "B" region green, "C" region cyan). Whilst none of these epitopes maps directly to the factor H recognition site they all lie sufficiently close that binding of the large antibody is likely to hinder recognition of factor H. The histidine sidechain at the polymorphic residue 402 of factor H is also shown in a CPK representation. Although this residue lies close to a secondary contact with the factor H binding protein (around residues 103 to 108 - see Figure 2) the evidence from SPR of the two polymorphic forms suggests that this is not a critical contact. This is also supported by the fact that this region of the complex (both the GNA loops and the factor H domain) is the most mobile region seen with significant variation in the way the two proteins contact one another seen at this point between the crystallographic idependent copies of the complex (Figure 4).
**Figure 6** - is the amino acid sequence of the factor H binding protein (SEQ ID NO: 6). This protein has the GenBank Accession No: AAF42204.

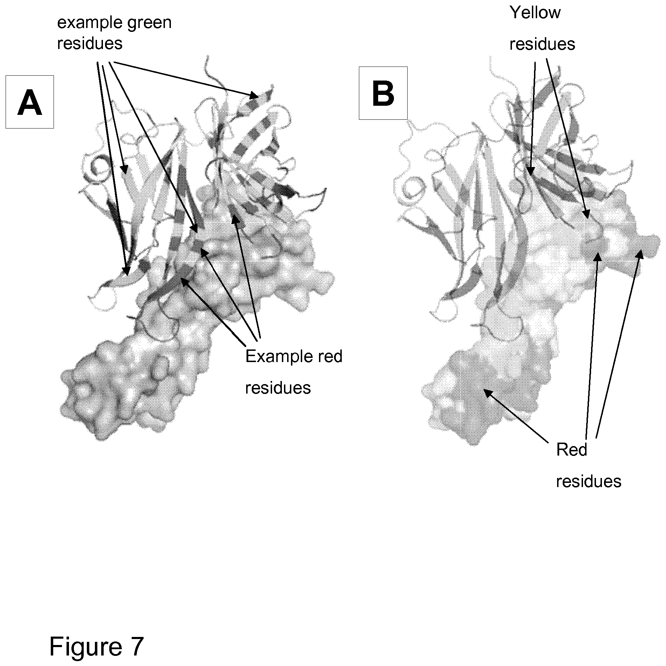

**Figure 7** - Structure of fHbp:fH67 complex. **Figure 7A** - fHbp shown as a cartoon (in a green residues conserved amongst families, red not conserved) and fH67 shown as a surface model. **Figure 7B** - Residues that vary between murine and human fH shown red; our work has shown yellow residues affect fH binding.

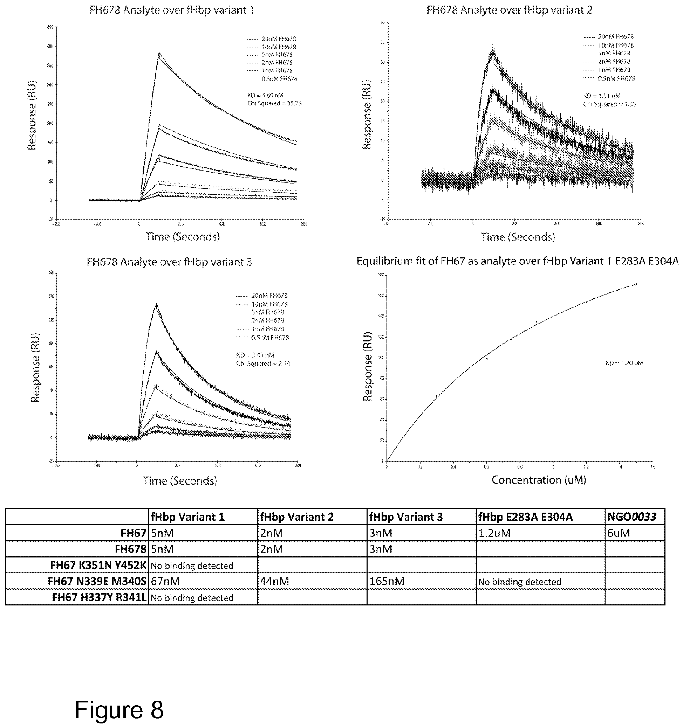

**Figure 8** **-** Binding of fHbp from variant 1, 2 and 3 with fH678 determined by SPR. Interactions with the gonococcal homologue, NGO0033 were also examined.

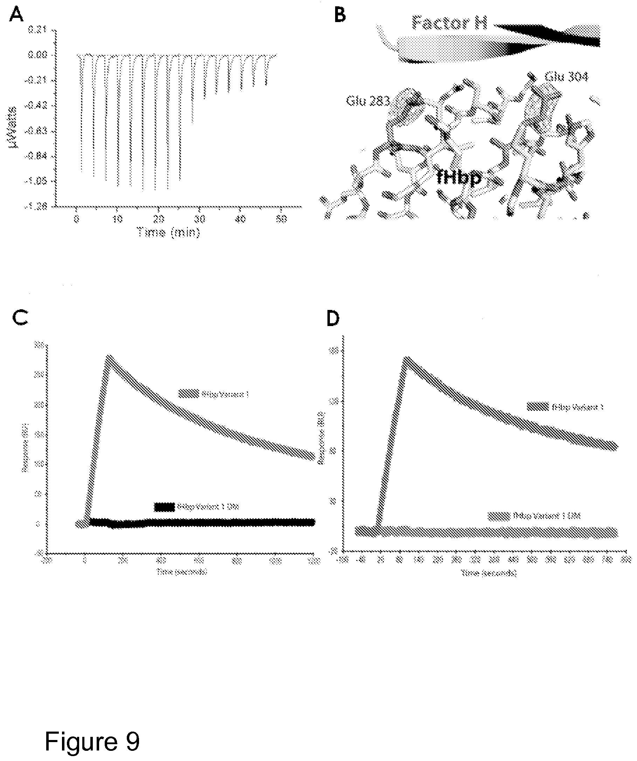

**Figure 9A****,** isothermal titration calorimetry confirms the fHbp/fH interaction is characterised by a nM KD in solution. **Figure 9B****,** the structure of the double Glu to Ala mutant fHbp confirms the only structural changes are in the mutated side chains (indicated by red difference electron density in a FO-FC, αC map. **Figure 9C****,** response from 5nM Factor H Domains 5, 6 and 7 flowed over WT and mutant fHbp (top data line = fHbp Variant 1, bottom data line =fHbp Variant 1 DM).. **Figure 9D****,** response from 20nM intact Factor H flowed over WT and mutant fHbp (top data line = fHbp Variant 1, bottom data line =fHbp Variant 1 DM).

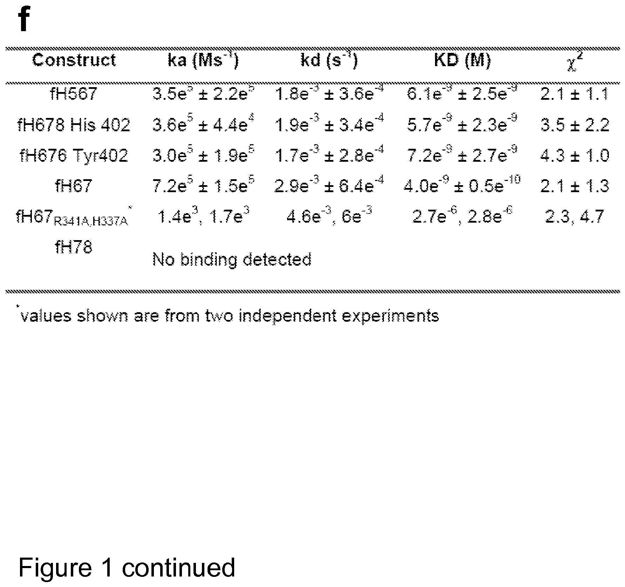

The function of the factor H binding protein, the sequence of which is given in Figure 1, has previously been studied by subdividing the protein into a series of regions, termed "A", "B" and "C", covering the entire extracellular domain (Giuliani et al. (2005) Infect Immun 73, 1151-60). Western blot analysis demonstrates that all three regions are necessary for high affinity interactions between factor H and the factor H binding protein (Figure 1a), implying that the factor H binding protein has an extended recognition site for factor H across its entire surface.

To identify which of the 20 CCP domains of factor H mediate the interaction with the factor H binding protein the following techniques were used: far Western analysis; FACS analysis (Figure 1b); and Surface Plasmon Resonance (Figure 1c). The results obtained demonstrate that the key region of factor H recognised by the factor H binding protein is the sixth domain, CCP6 and that constructs containing CCP6 were capable of inhibiting the factor H binding protein-dependent interaction between factor H and *N. meningitidis* (Figure 1d). Quantification of the interaction demonstrates that the dissociation constant is ~ 5nM (Figures 1c, 1e and 1f) for any construct containing CCP6. This interaction was not dissociated by high salt (1M NaCl, not shown) or pH ranging from 4 to 8 (not shown), providing additional support for the high affinity nature of the binding event.

With reference to Figure 9A-D, surface plasmon resonance demonstrates that the fHbp double mutant binds Factor H domains 6 & 7 with a two orders of magnitude higher KD. The mutant also binds a longer construct (consisting of domains 5, 6 and 7) and full length Factor H (purified from serum) much more weakly than wild-type fHbp.

Crystal structures of the complex between CCPs 67 of factor H (known hereafter as fH67) and the extracellular portion of the factor H binding protein comprising regions A, B and C were obtained, and models for the factor H binding protein and fH67 were built and refined to 2.35 A in two crystal forms with a total of seven independent copies of the complex (Figures 2a, b and c). As can be seen in Figure 2a the extracellular portion of the factor H binding protein folds to form two β-barrels with the N-terminal barrel consisting of the "A" and part of the "B" regions, whilst the C-terminal barrel is composed of the rest of the "B" and the "C" regions. A search of structural databases with the N-terminal barrel revealed no close structural homologues, and the distinct topologies of the β-barrels (Figure 2c) suggests that they have not arisen by a gene duplication event.

The fH67:fHBP (the factor H:factor H binding protein) complex is held together by extensive interactions between both β-barrels of the factor H binding protein and factor H CCP6 (Figure 2b), this is consistent with the binding studies. In particular, the helical insertion into the second β-strand of the CCP6 (an unusual feature of this CCP domain) is centrally located in the complex. Analysis by the PISA server gives all seven independent complexes a significance score of 1.0 (i.e. extremely likely to be biologically significant) with the average surface area of the factor H binding protein buried in the complex (2860 ± 177Å2) greater than that buried in most antibody: antigen complexes. Furthermore, the ΔG predicted on the basis of the structure for the formation of the complex (-6 kcal/mol) is in good agreement with the affinity derived from binding studies (-11 kcal/mol, calculated from KD presented in Figure 1e), providing additional support for the physiological relevance of the crystallised complex. The interaction surface shows good shape complementarity with numerous electrostatic interactions (Figure 2c) including multiple hydrogen bonds and salt bridges. Consistent with fHbp:fH interaction studies which did not detect any differences in binding (Figure 1e, Figure 4 and Figure 5) between the factor H 402His/Tyr isoforms associated with age related macular degeneration. His402 is only peripherally involved in the interaction with the factor H binding protein and contacts the flexible loop between strands 1 and 2 of the N-terminal β-barrel of the factor H binding protein (Figure 4).

To further probe the interaction surface doubly mutated/substituted forms of both proteins were generated, in which charged side chains were replaced by the small hydrophobic residue Alanine (A). In fH67 the mutations made were R341A and H337A, and in the factor H binding protein the mutations made were E283A and E304A. Using SPR to study the interactions between the mutated proteins and their wild-type partners revealed that the affinity of both mutant forms was reduced by more than two orders of magnitude so that almost no interaction could be seen at analyte concentrations (~ 50 nM) around ten times the wild-type KD (Figure 3a). At a thousand-fold higher analyte concentration (*µ*M range) the mutant forms of both proteins did interact with the wild-type partners: fHbpE283A,E304A retained a qualitatively similar on-rate but an increased off-rate with respect to wild-type whilst fH67R341A,H337A was more similar to the wild-type interaction in its off-rate but had a vastly reduced on-rate (Figure 1e).

### fHbp from different families share binding characteristics

Strains of N. meningitidis express a single fHbp belonging to one of three variant families, variant 1, variant 2 and variant 3. Our work has focussed on fHbp from variant 1, since this is the most prevalent family amongst disease isolates. Fig 7 shows the sequence variation amongst the three families mapped onto the structure of the complex and demonstrates that, although the level of sequence conservation is high (> 60% identical), there is significant variation around the fH-binding site. We therefore determined whether the mode of fH recognition is conserved between the different fHbp families and whether the same fHbp and fH residues are key to binding. This is essential information to generate reduced binding variants of fHbp to evaluate as vaccines.

To this end we have cloned, expressed, and purified fHbp from variant 2 and variant 3, and begun to characterise their interactions with fH (Fig. 8). Our SPR studies to date indicate that the modes of recognition are similar; affinities are of the same order of magnitude, and mutation of the same residues in fH has a dramatic effect on binding of fHbps from all families (not shown). Of note, while the Neisseria gonorrhoeae fHbp homologue (NGO0033) is closely related to variant 3 (91% amino acid identity), the purified protein fails to interact with fH to any appreciable extent (Fig. 8).

### The fHbpE283A,E304A mutant retains its atomic structure

A potential explanation for the failure of the fHbpE283A,E304A mutant (Double mutant, DM) to bind to fH67 with a dissociation constant (KD) in the nanomolar (nM) range is that the amino acid changes disrupt the overall structure of the protein. To address this, the crystal structure of the fHbpE283A,E304A mutant in complex with fH67 was determined.

The results confirmed that the only structural changes to fHbp were to the mutated side chains (Fig. 9B). Additionally we determined the relevance of these mutations in binding to fH5,6,7 and full length fH (Fig. 9C and D). Concentrations of fH in the 10nM range could be seen to interact with the wild-type protein suggesting that the affinity for the full length fH is in the same range as that for the smaller fragments (e.g. fH5,6,7). However, the binding affinity of the fHbpE283A,E304A protein is dramatically reduced compared to the wild-type protein.

Mapping of published epitopes of the factor H binding protein that elicit bactericidal antibodies (Giuliani et al. (2005) Infect Immun 73, 1151-60; Cantini et al. (2006) J Biol Chem 281, 7220-7) onto the structure demonstrates that none of the sites characterised to date lie directly in the factor H recognition site. However, epitopes recognised by antibodies that affect factor binding are located around the edge of the recognition site and so are likely to inhibit factor H binding by steric hindrance (Figure 5).

### fHbp single and double mutants retain their immunogenicity

To examine the impact of the E283A and E304A amino acid changes, proteins from fHbp variant 1 with these substitutions in isolation (i.e. fHbp_{E283A}, and fHbp_{E304A}) or in combination (fHbp_{E283A,E304A}) were expressed, and purified. The wild-type fHbp and these proteins were used to immunise groups of mice (5/group), and the presence of bactericidal antibodies in immune sera was determined. Results (Table 1) are expressed as the reciprocal of the highest dilution of serum that gave 50% or more killing of wild-type serogroup B *N. meningitidis* (strain H44/76). Pre-immune sera gave no killing.

**Table 1 Serum bactericidal antibody (SBA) titres in animals immunised with wild-type or mutant fHbp**

| Sera raised against | SBA titre against H44/76 |
|---|---|
| fHbp | 64 |
| fHbp_{E283A,E304A} | 64 |
| fHbp_{E283A} | 256 |
| fHbp_{E304A} | 64 |

The results show that the mutant proteins have at least equal ability to elicit SBA titres against serogroup B *N. meningitidis* as the wild-type protein.

### Methods

**Western blot analysis:** Protein samples were separated by SDS-PAGE, then transferred to PVDF membranes at 15 V for 60 minutes. The membranes were blocked in 5% milk in PBS at 4°C overnight, rinsed once in PBSTM (PBS, 0.05% Tween20, 0.5% skimmed milk), then incubated with primary antibodies or a source of fH for 1 to 2 hrs at room temperature. After three washes in PBSTM, membranes were incubated with goat α-human fH pAbs (at a 1:1000 final dilution) or murine α-fHbp pAbs (at a 1:10,000 final dilution) in PBS. Binding was detected with an α-rabbit peroxidase conjugated IgG (Dakocytomation) was used at 1:500 dilution.

**Flow cytometry analysis:** A total of 10⁸ *N. meningitidis* were incubated with 10 *µ*l of purified fH (2 µM) or CCPs (final concentration, 3 mM) for 30 minutes at 37°C. After three washes with PBS-0.1%Tween20 (PBST), bacteria were incubated for 30 min with a mAb MRC OX241 or with α-human factor H pAbs in PBS. After three washes in PBST, cells were incubated with a secondary, FITC-conjugated antibody for 30 minutes in the dark at 4°C. Factor H bound to meningococci was detected by flow cytometry using a FACS Calibur analyser (Becton Dickinson). For inhibition assays, full length factor H was incubated with bacteria together with increasing concentrations of CCPs 67 as indicated.

**Expression of proteins and purification of the complex:** factor H binding protein and truncated versions of the protein were expressed in *E. coli* BL21(DE3) host cells as polyhistidine-tagged fusion proteins at their C terminus (Masignani et al (2003) J Exp Med Vol 197, No 6, 789-97). Cultures of the strains were grown to mid-log phase and expression induced with isopropyl-D-thiogalactoside (IPTG, 1 mM final concentration). Proteins were purified by affinity chromatography on a His-Trap column according to the manufacturer's instructions (GE Healthcare), and eluted from columns with 250 mM imidazole. Factor H constructs were expressed in *E. coli* as previously described (with refolding to allow correct formation of the four disulphide bonds within the two CCP fragment) and purified either by heparin column affinity purification or by size exclusion chromatography. Following mixing of fHbp with fH67 (at a large molar excess), the complex was purified by size exclusion chromatography (Superdex S-200, GE Healthcare Inc., buffer Tris pH 7.5, 150mM NaCl) where the strong interaction seen between the uncomplexed fH67 and the column matrix ensured good separation of the peak containing the complex and that containing the excess fH67. Presence of both proteins in the putative complex peak was confirmed by SDS-PAGE and mass spectrometry (data not shown).

**Site-directed mutagensis:** Double mutants were generated in a sequential fashion using the QuikChange site-directed mutagenesis kit (Stratagene).

The R341A, H337A double mutant form of fH67 was generated using the following primers:
H337A forward, 
5’AAACATGGAGGTCTATATGCTGAGAATATGCGTAGACCATACTT
TCC3’ (SEQ ID NO: 2);
H337A reverse, 
5'TTTGTACCTCCAGATACGACTCTTATACGCATCTGGTATGAAAG
G3'(SEQ ID NO: 3).
R341A forward, 
5’GGTCTATATCATGAGAATATGGCTAGACCATACTTTCCAGTAGC
3’(SEQ ID NO: 4);
R341A reverse,

5'CCAGATATAGTACTCTTATACCGATCTGGTATGAAAGGTCATCG
3'(SEQ ID NO: 5).

Introduction of the mutation was confirmed by sequencing. Recombinant mutant fH67 fragments were expressed and refolded from inclusion bodies as previously described (Prosser et al (2007) Acta Crystallogr Sect F Struct Biol Cryst Common Jun 1:63(PT6):480-3).

For generation of the E283A, E304A factor H binding protein the primers used were:
E283A forward 5' ACAACCAAGCCGCGAAAGGCAGTTAC 3';
E283A reverse, 5' GTAACTGCCTTTCGCGGCTTGGTTGT 3';
E304A forward TGCCGGCAGCGCGGCAGTGAAAACCG 5';
E304A reverse 5' CGGTTTTCACTGCCGCGCTGCCGGCA 3'
and the protein expressed and purified as described above.

**Surface Plasmon Resonance:** Either the factor H binding protein or the factor H constructs were coupled to the sensor surface (CM5 chips) using standard amine coupling protocols. The appropriate ligand was then flowed in the fluid phase at a flow rate of 40 µl/min. The strong interaction was not disrupted using either high/low salt (0 to 3M NaCl) or extreme pH (range 4 to 8 tried) and extended dissociation time (60 minutes) was therefore used between successive injections. The data obtained with the larger factor H binding protein in the fluid phase could not be satisfactorily fit using a simple 1:1 model (presumably due either to issues of movement of the larger protein into/out of the chip matrix or steric hindrance in the factor H binding site introduced by coupling of the smaller protein to the chip surface), crude analysis of near-equilibrium values suggested a KD in the tens to hundreds of nM range (depending on the factor H construct used). Once the interaction was reversed (with fH on the chip) the data could be satisfactorily fit (Figure 1f) using a 1:1 Langmuir model with χ² of ~ 3 revealing that the true KD for the constructs containing CCP6 was of the order of 5nM (see Figure 1f). The fH67 and the factor H binding protein double mutants were also analysed with the fHbp partner coupled to the chip surface (fHbp_{WT} ~ 800 RU coupled on channel 1, fHbp_{E283A,E304A} ~ 1500 RU coupled on channel 2). fH67 (WT and mutant) were then flown over at a concentration of 50µM (~ 10 x the native K_{D}) revealing little or no interaction from the mutants with their WT partner compared to the magnitude of interaction seen with the WT pairing. To calculate the affinity of fH67_{R341A,H337A} for fHbp_{WT} a series of injections (in duplicate) spanning the concentration range 250nM to 20µM was used and fit using the BiaEvaluation software to give the values shown in Figure 1f.

**Crystallisation and X-ray data collection:** The crystals were grown using the sitting drop vapour diffusion method from 0.2µl complex + 0.2µl mother liquor drops (complex at 4.5 mg/ml calculated using an assumed extinction coefficient of 2.2, mother liquor optimised around JCSG-plus condition 15, 20% polyethylene glycol 6000 MW, 0.1M Bicine pH 9.0). Harvesting of crystals and SDS-PAGE confirmed that both fH67 and the factor H binding protein were present (data not shown). Crystals (usually 50 x 10 x 5 µm) were flash frozen in liquid nitrogen following cryoprotection with 15% ethylene glycol. Data were collected at both the ESRF and Diamond by the rotation method using either and oscillation range of 0.5 or 1 degree with the crystal kept at 120K. The Native P1 data were collected at beamline ID14eh4 (ESRF) with lambda 0.9523 Å, the Native C2 and Pt sets were collected at beamline I03 (Diamond) with lambda 0.9814 Å, the Hg set at beamline BM14 (ESRF) with lambda 1.003 Å and the S-SAD at beamline ID29 (ESRF) with lambda 1.8Å. Data were integrated and scaled using xia2 with the -3d option to force use of program XDS4 for integration and program Scala5 for inter-frame scaling.

**Structure Solution and Refinement:** The structure of the fh67/fHbp complex was solved in the C2 crystal form by a combination of molecular replacement with the structure of fh67 (taken from the earlier structure of fH678 in complex with sucrose octasulphate, SOS, PDBID:2UWN) and MIRAS methods using Pt, Hg derivatives and the anomalous scattering from 31 of the 33 sulphurs present in the C2 asymmetric unit. Three copies of fH67 were found using MolRep7 (CCP4 Program Suite) and the two CCP domains rigid body refined using Buster-TNT without modelling of missing atoms. These phases were then incorporated into a SHARP phasing job and used to locate the heavy atom sites in the other crystals (6 Pt, 1Hg and 31 S). The heavy atom model was refined in SHARP (with B factor fixed to the Wilson value for each crystal) using data to 3.2Å and the phases generated combined with those from the molecular replacement within SHARP to generate phases with a figure of merit of 0.54 (0.35 for the highest resolution shell 3.3-3.2A). The phasing powers for the derivatives were (anomalous/isomorphous) 0.7/2.4 for the Pt, 0.2/0.3 for the Hg and 0.3/0.0 for the anomalous sulphur signal. The map obtained using these phases was solvent flattened within SHARP and averaged in DM using three separate operators for the factor H binding protein, Factor H CCP6 and factor H CCP7 regions. The NMR model (Cantini et al (2006) J Biol Chem Mar 17:281(11):7220-7) for the C-terminal barrel of the factor H binding protein could not be placed in this density (the strands were seen to be distorted) so a model was built by hand using program Coot. Rebuilding and refinement using data in the range 40 to 2.35 Å proceeded using Coot and Buster-TNT with non crystallographic symmetry used to restrain the core of each domain whilst more variation was allowed between the loop region until the R/Rfree were 25.5/27.1 with no residues in the disallowed regions of the Ramachandran plot (94.6% in the most favoured, as determined by MolProbity). The model contains all residues of fH67 and all but five residues at the N-terminus of the factor H binding protein and the six histidines of the affinity tag which are disordered in all copies of the molecule. Molecular replacement of the individual domains (factor H CCP6 and CCP7 and the factor H binding protein) using program MolRep allowed solution of the P1 crystal form (four independent copies of the complex in a different packing arrangement). Minor rebuilding of the loop regions and refinement (90 to 2.35Å) using Coot/Buster-TNT led to R/Rfree of 23.2/26.3 once again with no residues in the disallowed regions of the Ramachandran plot (95.3% in the most favoured). Structural figures are drawn using program **PyMol.**

### Immunisation protocol

Seven-week old BALB/C mice were immunised by the subcutaneous route with purified fHbp or with mutant fHbp subcutaneously with Freunds' adjuvant on days 0 and 10. Each animal received 25 micrograms of protein in PBS with an equal volume of adjuvant to a total volume of 200 microliters. On day 14, animals were sacrificed and sera were collected from mice and pooled then aliquoted and stored at -80°C.

### Serum bactericidal activity (SBA)

The serogroup B Neisseria meningitidis strain H44/76 was grown overnight on solid media, harvested into PBS, and the number of colony forming units quantified. Serial dilutions of sera were added to bacteria (104 CFU in SBA buffer) and baby rabbit complement (Pelfreeze, 1 in 8 final dilution) for 1 hr and the number of surviving bacteria determined by plating to solid media.

## Claims

1. An immunogenic composition for use in the treatment or prevention of infection or disease caused by *Neisseria meningitidis* and/or *Neisseria gonorrhoeae,* comprising at least one modified factor H binding protein, wherein the composition is capable of eliciting an immune response when administered to a human or non-human animal, wherein the modified factor H binding protein has an amino acid sequence having at least 70% sequence identity to the amino acid sequence of Seq ID No:1, and has an alanine at position number 283 as defined in Seq ID No: 1 instead of glutamic acid, which results in no factor H binding, or at least 5 fold less factor H binding compared with the factor H binding protein of Seq ID No:1.

2. The immunogenic composition for use according to claim 1 wherein the factor H binding protein is derived from *Neisseria meningitidis* or *Neisseria gonorrhoeae.*

3. The immunogenic composition for use according to claim 1 comprising a further one or more antigens in addition to the modified factor H binding protein.

4. The immunogenic composition for use according to claim 1 wherein the composition is capable of eliciting a protective immune response to *Neisseria meningitidis* and/or *Neisseria gonorrhoeae.*

5. Use of a modified factor H binding protein for the preparation of a medicament for the treatment or prevention of infection or disease caused by *Neisseria meningitidis* and/or *Neisseria gonorrhoeae,* wherein the modified factor H binding protein is capable of eliciting an immune response when administered to a human or non-human animal having an amino acid sequence having at least 70% sequence identity to the amino acid sequence of Seq ID No:1, and having an alanine at position number 283 as defined in Seq ID No: 1 instead of glutamic acid, which results in no factor H binding, or at least 5 fold less factor H binding compared with the factor H binding protein of Seq ID No:1.

6. Use of the modified factor H binding protein according to claim 5, wherein the factor H binding protein is derived from *Neisseria meningitidis* or *Neisseria gonorrhoea.*

7. Use of the modified factor H binding protein according to claim 5, wherein the composition is capable of eliciting a protective immune response to *Neisseria meningitidis* and/or *Neisseria gonorrhoeae.*

## Patentansprüche

1. Immunogene Zusammensetzung zur Verwendung bei der Behandlung oder Vorbeugung von Infektion oder Krankheit, die durch *Neisseria meningitidis* und/oder *Neisseria gonorrhoeae* verursacht wird, umfassend mindestens ein modifiziertes Faktor-H-Bindungsprotein, wobei die Zusammensetzung in der Lage ist, eine Immunantwort auszulösen, wenn sie einem Menschen oder einem nichtmenschlichen Tier verabreicht wird, wobei das modifizierte Faktor-H-Bindungsprotein eine Aminosäuresequenz mit mindestens 70 % Sequenzidentität zu der Aminosäuresequenz von Seq ID No: 1 aufweist, und ein Alanin an Position Nummer 283, wie in Seq ID No: 1 definiert, anstelle von Glutaminsäure aufweist, was zu keiner Faktor-H-Bindung oder zu einer mindestens 5-fach geringeren Faktor-H-Bindung im Vergleich zu dem Faktor-H-Bindungsprotein von Seq ID No: 1 führt.

2. Immunogene Zusammensetzung zur Verwendung gemäß Anspruch 1, wobei das Faktor-H-Bindungsprotein von *Neisseria meningitidis* oder *Neisseria gonorrhoeae* abgeleitet ist.

3. Immunogene Zusammensetzung zur Verwendung gemäß Anspruch 1, umfassend ein weiteres Antigen oder mehrere Antigene zusätzlich zu dem modifizierten Faktor-H-Bindungsprotein.

4. Immunogene Zusammensetzung zur Verwendung gemäß Anspruch 1, wobei die Zusammensetzung in der Lage ist, eine schützende Immunantwort gegen *Neisseria meningitidis* und/oder *Neisseria gonorrhoeae* auszulösen.

5. Verwendung eines modifizierten Faktor-H-Bindungsproteins zur Herstellung eines Medikaments zur Behandlung oder Vorbeugung einer Infektion oder Krankheit, die durch *Neisseria meningitidis* und/oder *Neisseria gonorrhoeae* verursacht wird, wobei das modifizierte Faktor-H-Bindungsprotein in der Lage ist, eine Immunantwort auszulösen, wenn es einem Menschen oder einem nichtmenschlichen Tier verabreicht wird, aufweisend: eine Aminosäuresequenz mit mindestens 70 % Sequenzidentität zu der Aminosäuresequenz von Seq ID No: 1, und ein Alanin an Position Nummer 283, wie in Seq ID No: 1 definiert, anstelle von Glutaminsäure, was zu keiner Faktor-H-Bindung oder zu einer mindestens 5-fach geringeren Faktor-H-Bindung im Vergleich zu dem Faktor-H-Bindungsprotein von Seq ID No: 1 führt.

6. Verwendung des modifizierten Faktor-H-Bindungsproteins gemäß Anspruch 5, wobei das Faktor-H-Bindungsprotein von *Neisseria meningitidis* oder *Neisseria gonorrhoea* abgeleitet ist.

7. Verwendung des modifizierten Faktor-H-Bindeproteins gemäß Anspruch 5, wobei die Zusammensetzung in der Lage ist, eine schützende Immunantwort gegen *Neisseria meningitidis* und/oder *Neisseria gonorrhoeae* auszulösen.

## Revendications

1. Composition immunogène pour utilisation dans le traitement ou la prévention d'une infection ou d'une maladie provoquée par *Neisseria meningitidis* et/ou *Neisseria gonorrhoeae,* comprenant au moins une protéine de liaison du facteur H modifiée, dans laquelle la composition est capable de provoquer une réponse immunitaire lorsqu'elle est administrée à un être humain ou animal non-humain, dans laquelle la protéine de liaison du facteur H modifiée a une séquence d'acides aminés ayant au moins 70 % d'identité de séquence avec la séquence d'acides aminés de Seq ID No : 1, et a une alanine à la position numéro 283 telle que définie dans Seq ID No : 1 au lieu de l'acide glutamique, ce qui entraîne une absence de liaison du facteur H, ou une liaison du facteur H au moins 5 fois inférieure par comparaison à la protéine de liaison du facteur H de Seq ID No : 1.

2. Composition immunogène pour utilisation selon la revendication 1, dans laquelle la protéine de liaison du facteur H est dérivée de *Neisseria meningitidis* ou de *Neisseria gonorrhoeae.*

3. Composition immunogène pour utilisation selon la revendication 1, comprenant un ou plusieurs antigènes supplémentaires en plus de la protéine de liaison du facteur H modifiée.

4. Composition immunogène pour utilisation selon la revendication 1, dans laquelle la composition est capable de provoquer une réponse immunitaire protectrice contre *Neisseria meningitidis* et/ou *Neisseria gonorrhoeae.*

5. Utilisation d'une protéine de liaison du facteur H modifiée pour la préparation d'un médicament destiné au traitement ou à la prévention d'une infection ou d'une maladie provoquée par *Neisseria meningitidis* et/ou *Neisseria gonorrhoeae,* dans laquelle la protéine de liaison du facteur H modifiée est capable de provoquer une réponse immunitaire lorsqu'elle est administrée à un être humain ou animal non-humain ayant une séquence d'acides aminés ayant au moins 70 % d'identité de séquence avec la séquence d'acides aminés de Seq ID No : 1, et ayant une alanine à la position numéro 283 telle que définie dans Seq ID No : 1 au lieu de l'acide glutamique, ce qui entraîne une absence de liaison du facteur H, ou une liaison du facteur H au moins 5 fois inférieure par comparaison à la protéine de liaison du facteur H de Seq ID No : 1.

6. Utilisation de la protéine de liaison du facteur H modifiée selon la revendication 5, dans laquelle la protéine de liaison du facteur H est dérivée de *Neisseria meningitidis* ou de *Neisseria gonorrhoea.*

7. Utilisation de la protéine de liaison du facteur H modifiée selon la revendication 5, dans laquelle la composition est capable de provoquer une réponse immunitaire protectrice contre *Neisseria meningitidis* et/ou *Neisseria gonorrhoeae.*
